**Europäisches Patentamt**

**European Patent Office**

**Office européen des brevets**

(11) Veröffentlichungsnummer: **0 209 726**
**A2**

## EUROPÄISCHE PATENTANMELDUNG

(21) Anmeldenummer: 86108283.2

(22) Anmeldetag: 18.06.86

(51) Int.Cl.⁴: **C 08 L 89/06**
**A 61 K 47/00, A 61 L 15/00**

---

(30) Priorität: 25.06.85 DE 3522626

(43) Veröffentlichungstag der Anmeldung:
**28.01.87 Patentblatt 87/5**

(84) Benannte Vertragsstaaten:
**AT BE CH DE FR GB IT LI LU NL SE**

(71) Anmelder: **Merz & Co. GmbH & Co.**
**Eckenheimer Landstrasse 100-104**
**D-6000 Frankfurt/M. 1(DE)**

(72) Erfinder: **Beutler, Rolf-Dieter, Dr. Dipl.-Chem.**
**Frauensteinstrasse 3**
**D-6000 Frankfurt/Main(DE)**

(72) Erfinder: **Ebinger, Jürgen, Dipl.-Ing.**
**A.d.Kirschäcker 7a**
**D-6239 Eppstein(DE)**

(72) Erfinder: **Lindner, Helmut**
**Gassbacher Weg 40**
**D-6149 Graseflenbach 1(DE)**

(74) Vertreter: **Bell, Hans Chr. et al,**
**Bell, Wolff und Bell, Rechtsanwälte Adelonstrasse 58**
**Postfach 80 01 40**
**D-6230 Frankfurt am Main 80(DE)**

---

(54) Löslicher Kollagen-Schwamm.

(57) Die Erfindung betrifft einen Kollagen-Schwamm, der eine Denaturierungstemperatur von mindestens 37°C hat, unvernetzt ist und in einer seiner beiden Ausführungsformen in wäßrigem Medium eine optisch leere Lösung bildet. Er eignet sich im Vergleich zu den bisher bekannten Kollagen-Schwämmen besonders als Trägermaterial für pharmazeutische und kosmetische Wirkstoffe, weil er eine höhere Denaturierungstemperatur aufweist als diese und sich in Berührung mit Körperflüssigkeit wieder auflöst. Der erfindungsgemäße Kollagen-Schwamm wird aus einer optisch leeren Kollagenlösung hergestellt, indem man mittels Neutralsalzen fällt, löst und wieder fällt und dann das Kollagen ohne Entfernung des Fällungssalzes einem Gefriervorgang unterwirft. Der Lösung können auch säuregequollene Kollagene und/oder Cellulose oder Cellulosederivate zugesetzt werden, wodurch sich die physikalischen Eigenschaften des Kollagen-Schwamms beeinflussen lassen.

EP 0 209 726 A2

Unsere Nr. 25 135                                    Wo/br

Merz + Co. GmbH & Co.
Eckenheimer Landstr. 100-104
6000  Frankfurt/Main 1

<u>Löslicher Kollagen-Schwamm</u>

Gegenstand der Erfindung ist ein Kollagen-Schwamm, der sich in wäßriger Lösung oder in Berührung mit Körperflüssigkeiten wieder auflöst und der eine Denaturierungstemperatur von mindestens 37°C aufweist. Ein solcher Kollagen-Schwamm ist insbesondere geeignet als Trägermaterial für pharmazeutische und kosmetische Wirkstoffe.

Trägermaterialien aus Kollagen in schwammartiger bzw. filzartiger Struktur sind bereits bekannt. Sie haben jedoch eine zu niedrige Denaturierungstemperatur und lösen sich vor allem in wäßrigem Medium oder in Körperflüssigkeiten nicht wieder auf. Diese Kollagen-Trägermaterialien gemäß dem Stand der Technik werden in der Regel mittels Vernetzungsreaktionen, z.B. mit Aldehyden, hergestellt.

Bei solchen vernetzten Kollagenprodukten als Wirkstoffträger besteht der Nachteil, daß sie im Körper Entzündungen, Abwehrreaktionen oder die Bildung von Fremdkörperriesenzellen verursachen können. Das durch die
Vernetzung erhaltene Kollagenprodukt ist unlöslich in
Bezug auf physiologische Lösungen sowie Körperflüssigkeiten, so daß ein manuelles Entfernen aus Körperhöhlen
(z.B. Vaginalraum) nach der Applikation erforderlich
ist.

Bei diesen bisher beschriebenen Kollagenprodukten
besteht ferner der Nachteil, daß durch die Trocknung
des Kollagens irreversible Vernetzungen entstehen und
somit, selbst wenn als Ausgangslösung lösliches Kollagen
eingesetzt wird, ein Verlust oder eine erhebliche
Minderung der Löslichkeit auftritt. Bisher bekannte
Kollagenprodukte besitzen nicht die Fähigkeit, durch
Wasserzusatz bzw. Körperflüssigkeiten erneut eine
optisch leere Kollagenlösung zu bilden, und sie müssen
somit nach der Applikation manuell entfernt werden, was
z.B. bei intravaginaler Anwendung einen erheblichen
Nachteil darstellt (Toxic-Schock-Syndrom). Diese Nachteile werden durch den erfindungsgemäßen Kollagen-
Schwamm vermieden.

Der erfindungsgemäße Kollagen-Schwamm wird durch das
nachstehend beschriebene Verfahren hergestellt. Man
geht vorzugsweise aus von Häuten junger Säugetiere,
wobei die Häute zunächst auf übliche Art gereinigt werden.
Nach dem Reinigen erfolgt ein Extrahieren des löslichen
Kollagens von den unlöslichen Anteilen der Häute
mittels Säuren, vorzugsweise organischen Säuren mit niederem Molekulargewicht, das besonders bevorzugt
nicht höher als 200 ist. Bei der Herstellung dieser
Ausgangslösung ist durch Steuerung der Extraktion bzw.
der Konzentration des Kollagens und mittels polarimetrischer Messungen darauf zu achten, daß eine echte,
d.h. optisch leere Lösung erhalten wird. Das in dieser
Lösung enthaltene Kollagen hat eine Denaturierungstemperatur von mindestens 37°C, vorzugsweise 39 bis
45°C.

Aus der Lösung wird - gegebenenfalls unter mehrfacher
Wiederholung - das Kollagen mit Neutralsalzen, vorzugsweise Natriumchlorid oder Natriumsulfat, gefällt. Die
Salzkonzentration - bezogen auf das Volumen der Gesamtlösung - beträgt vorzugsweise 3 bis 5 Gew.-%. Es ist
erfindungsgemäß wichtig, daß das Fällungssalz oder
ein Teil desselben bei dem gefällten Kollagen verbleibt,
es also nicht durch Dialyse oder sonstige Verfahren
davon entfernt wird. Man erhält so eine salzhaltige
Zwischenphase von fester oder annähernd fester Konsistenz, die einen Kollagengehalt von ca. 20 bis
40 Gew.-% aufweist.

Diese Zwischenphase wird wieder aufgelöst zu einem
Kollagengehalt von weniger als 20 bis 40 Gew.-%,
vorzugsweise 0,1 bis 3 Gew.-%. Die so erhaltene Lösung
wird in mehreren Schritten einer Gefriertrocknung
unterzogen. Zunächst erfolgt eine Schockgefrierung,
wobei die Ausbildung von mit bloßem Auge erkennbarer
Eiskristalle mit üblichen Mitteln, wie z.B. Rühren
oder Schütteln der Lösung, verhindert wird. Darauf wird weiter auf ca. -25°C bis -40°C abgekühlt und
einige Stunden stehengelassen, bis sich in der Kollagenphase Fasern oder Fibrillen bilden. Schließlich erfolgt
die eigentliche Gefriertrocknung, d.h. unter Anwendung
von Vakuum.

Man erhält so einen Kollagen-Schwamm, der erst bei
mindestens 37°C, vorzugsweise 39 bis 45°C, denaturiert,
der unvernetzt ist und in wäßrigem Medium eine optisch
leere Lösung bildet, welche also in polarisiertem Licht
keine Brechung hervorruft. Die Dichte dieses Kollagen-
Schwamms beträgt vorzugsweise 15 bis 35 mg/cm³. Je
nach Form des Gefäßes, in welchem die Gefriertrocknung
durchgeführt wird, können verschiedene Formen (z.B.
Zylindersegment, Kugelsegment) erhalten und kann das
Kollagenprodukt aufgrund seiner schwammähnlichen
Struktur leicht in jede gewünschte Größe oder Form
gebracht werden, so wie es für den jeweiligen Anwendungszweck erforderlich ist.

Außerdem können die physikalischen Eigenschaften des
Schwamms (Festigkeit, Rückstellkraft) über den Ausgangsgehalt an löslichem Kollagen vor der Gefriertrocknung
gesteuert werden, so daß auch durch eine solche
Beeinflussung der physikalischen Eigenschaften besonders
vorteilhafte Ausführungsformen für bestimmte Applikationen geschaffen werden können.

Die physikalischen Eigenschaften können auch dadurch gesteuert werden, daß man dem eine optisch leere Lösung bildenden Kollagen säuregequollene Kollagene, die als solche keine optisch leere Lösung bilden, und/oder Cellulose oder Cellulosederivate zusetzt. Dies geschieht zweckmäßig nach der Fällung mittels Neutralsalzen in der danach wieder gebildeten Lösung. Das Zusetzen kann aber auch schon in der Ausgangslösung vor dem Fällen erfolgen. Mit der Menge der zugesetzten vorgenannten Stoffe nimmt die Löslichkeit und Lösungsgeschwindigkeit des letztlich erhaltenen Kollagen-Schwamms ab, und er erhält eine größere Stabilität gegen äußere Einflüsse, wie Feuchtigkeit, Druck und Zug. Ein solches Produkt ist insbesondere dann erwünscht, wenn es nicht im Vaginalbereich, sondern topisch verwendet werden soll, wenn also eine längere Erhaltungsdauer des Kollagen-Schwamms erwünscht ist, wie z.B. zum Zwecke der Okklusion offener Wunden.

Es hat sich jedoch herausgestellt, daß im Verhältnis zu dem eine optisch leere Lösung bildenden Kollagen der Anteil des säuregequollenen Kollagens und/oder der Cellulose oder Cellulosederivate nicht größer als 65% sein sollte, weil sich oberhalb dieses Mengenbereichs ein letztlich unlöslicher Kollagen-Schwamm ergeben kann. Die Denaturierungstemperatur und die Dichte des Kollagen-Schwamms ändern sich durch die hier in Rede stehenden Zusätze nicht wesentlich.

Die Einarbeitung der pharmazeutischen oder kosmetischen Wirkstoffe kann auf übliche Weise in den fertigen Kollagen-Schwamm erfolgen. Vorzugsweise bringt man den Wirkstoff jedoch in eine der oben beschriebenen Kollagenlösungen ein, ganz besonders bevorzugt in
diejenige Lösung, die auf einen Kollagengehalt von
weniger als 20 bis 40 Gew.-% verdünnt ist und die dann
den Gefrierungsschritten unterworfen wird. Dieses
bevorzugte Verfahren erlaubt es, geringere Mengen Wirkstoff zu verwenden und führt außerdem zu einer besonders
homogenen Verteilung des Wirkstoffs im Kollagen-Schwamm.

Der Vorteil des erfindungsgemäßen Kollagen-Schwamms
besteht ganz allgemein darin, daß durch die Ausbildung
von Fibrillen oder Fasern eine feinporige, gleichmäßige,
schwammähnliche Kollagenstruktur aufgebaut wird, die
nach Kontakt mit Wasser bzw. Körperflüssigkeiten unter
Auflösung erneut eine optisch leere Kollagenlösung
bildet. Es ist somit also nicht mehr erforderlich, den
Kollagen-Schwamm nach der Applikation manuell zu
entfernen.

Weitere Vorteile sind:
Da außer dem erfindungsgemäßen Trägermaterial keine
weiteren Hilfsstoffe in das Präparat eingearbeitet
werden müssen, sind Unverträglichkeiten auf ein Minimum
reduziert.
Bei Anwendung in Körperhöhlen kann ohne vorheriges
Tränken mit Wasser appliziert werden, wodurch ein
Wirkstoffverlust durch Wasserkontakt vermieden und
die Kontaminationsgefahr reduziert wird.
Die Wirkstoffe werden vollständig freigesetzt,
da das Trägermaterial sich völlig auflöst.
Der lösliche Kollagen-Schwamm erlaubt eine wesentlich
geringere Menge von Wirkstoffen, z.B. Kontrazeptiva,
im Vergleich zu unlöslichem Trägermaterial.

Schließlich treten auch keine Probleme bei der Abfallbeseitigung auf.

Die Anwendungsmöglichkeiten für erfindungsgemäße,
mit Wirkstoffen versehene Kollagenschwämme sind vielfältig. So lassen sich z.B. mit Desinfizientien, Antiseptika, Wundbehandlungsmitteln, Antibiotika oder
Chemotherapeutika versehene erfindungsgemäße Kollagen-
Schwämme auf die Haut, die Schleimhaut oder Wunden
auflegen. Ganz besonders geeignet sind die mit den jeweiligen Wirkstoffen versehenen erfindungsgemäßen
Kollagen-Schwämme für das Einbringen in Körperhöhlen,
wie After, Scheide, Nase oder Ohr. Ganz besonders
bevorzugt ist der erfindungsgemäße Kollagen-Schwamm
als Trägermaterial für Kontrazeptiva zum Einbringen
in die Scheide.

Patentansprüche

1. Kollagen-Schwamm, dadurch gekennzeichnet, daß er
   eine Denaturierungstemperatur von mindestens 37°C
   hat, unvernetzt ist und seine Dichte 15 bis
   35 mg/cm³ beträgt.

2. Kollagen-Schwamm nach Anspruch 1, dadurch gekennzeichnet, daß er in wäßrigem Medium eine optisch
   leere Lösung bildet.

3. Kollagen-Schwamm gemäß Ansprüchen 1 oder 2,
   dadurch gekennzeichnet, daß er pharmazeutische oder
   kosmetische Wirkstoffe eingearbeitet enthält.

4. Verfahren zur Herstellung von Kollagen-Schwamm gemäß
   Ansprüchen 1 oder 2, ausgehend von aus Häuten junger
   Säugetiere gewonnener saurer, optisch leerer Kollagenlösung, gekennzeichnet durch folgende Verfahrensschritte

   a) Fällung des Kollagens aus der Lösung mittels
      Neutralsalz,
   b) erneutes Lösen auf einen niedrigeren Kollagengehalt,
   c) Schockgefrierung der erhaltenen salzhaltigen
      Zwischenphase unter Verhinderung der Ausbildung
      mit dem bloßen Auge erkennbarer Eiskristalle,
   d) weitere Abkühlung auf -25 bis -40°C bis zur Ausbildung von Fasern oder Fibrillen in der Kollagenphase,
   e) Gefriertrocknung,
   gegebenenfalls unter Zusatz von säuregequollenem

-9-

Kollagen und/oder Cellulose oder Cellulosederivaten in die Lösung.

5. Verfahren zur Herstellung von Kollagen-Schwamm gemäß Anspruch 3, dadurch gekennzeichnet, daß der Wirkstoff

a) mit dem festen, aus der Lösung isolierten Kollagen-Schwamm in Berührung gebracht oder
b) in die Kollagenlösung eingebracht wird.